# EUROPEAN PATENT APPLICATION

(11) **EP 1 260 588 A2**
(43) Date of publication of application: **27.11.2002**
(21) Application number: 02009802.6
(22) Date of filing: 30.04.2002
(51) Int. Cl.: C12P 13/00

(54) **Methods of producing agmatine and arginine decarboxylase**

(30) Priority: 25.05.2001 JP 2001157833
(71) Applicant: Ajinomoto Co., Ltd., Tokyo (JP)
(72) Inventor: Matsui, Hiroshi, Kusatsu, Shiga 525-0059 (JP); Mori, Yukiko, Ajinomoto Co., Inc., Kyushi Plant, Saga-gun, Saga-ken 840-2193 (JP); Kikuchi, Yoshimi, Ajinomoto Co., Inc., Kawasaki-ku, Kawasaki-shi, Kanagawa 210 (JP); Onishi, Norimasa, Ajinomoto Co., Inc., Kawasaki-ku, Kawasaki-shi, Kanagawa 210 (JP)
(74) Representative: Strehl Schübel-Hopf & Partner

(57) **Abstract**

A recombinant wherein an arginine decarboxylase gene (adi) was amplified and expressed is created by genetic engineering means, and the recombinant, or arginine decarboxylase or an arginine decarboxylase-containing material obtained from the recombinant, is used to decarboxylate arginine to produce agmatine.

SEQUENCE LISTING

<110> AJINOMOTO Co., LTD

<120> METHODS OF PRODUCING AGMATINE AND ARGININE
DECARBOXYLASE

<130> EPA-53906

<140>
<141>

<160> 5

<170> PatentIn Ver. 2.1

<210> 1

<211> 30

<212> DNA

<213> Artificial Sequence

<220>

<223> Description of Artificial Sequence: primer1

<400> 1

gaccatggct aaagtattaa ttgttgaaag                                    30

<210> 2'

<211> 28

<212> DNA

<213> Artificial Sequence

<220>

<223> Description of Artificial Sequence: primer2

<400> 2

ccggatccac gccttcagcg gaatagtg                                      28

<210> 3

<211> 28

<212> DNA

<213> Artificial Sequence

<220>

<223> Description of Artificial Sequence: primer 3

<400> 3

ccctgcagat cagtatcagc caaaaaaa 28

<210> 4

<211> 28

<212> DNA

<213> Artificial Sequence

<220>

<223> Description of Artificial Sequence: primer 4

<400> 4

ccgaattcac gtccatccca acaatgtt 28

<210> 5

<211> 28

<212> DNA

<213> Artificial Sequence

<220>

<223> Description of Artificial Sequence: primer 5

<400> 5

ccgcatgcgg cgatgcgtgt gaagaaaa 28

## Description

### FIELD OF THE INVENTION

This invention relates to a method of producing agmatine enzymatically (i.e. by enzyme raction) from arginine as one of amino acids as a starting material. More particularly, this invention relates to a method of producing agmatine at a practical level by using a recombinant that has amplified and expressed arginine decarboxylase gene.

### BACKGROUND OF THE INVENTION

In recent years, agmatine attracts attention as an intermediate for production of acyl agmatine having various fatty acids added thereto. The acyl agmatine is found to have an excellent surfactant action, and used particularly for use in a shampoo or the like because it is superior in flexibility and moisture retention after washing compared to general-purpose quaternary ammonium type cationic surfactants.

As industrial method of producing acyl agmatine at present, chemical synthesis methods are general, but in the chemical synthesis processes, there is a problem that the synthesis route is very complicated which make the resultant acyl agmatine expensive. Accordingly, there is demand for a method of producing agmatine by an easier method at lower costs.

On one hand, arginine decarboxylase produced by microorganisms is known to produce agmatine from arginine which is one of amino acids as one substrate, as shown in the following reaction formula.

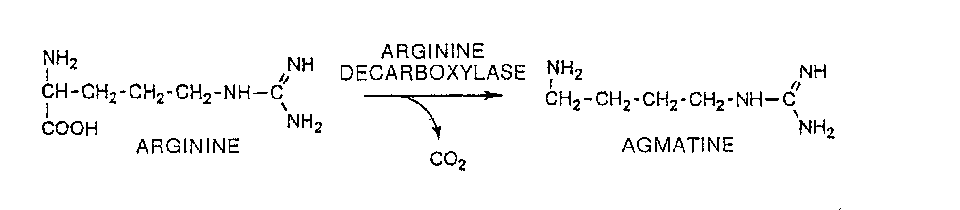

As such microorganisms producing arginine decarboxylase, the genus Escherichia including E. coli and the genus Salmonella (J. Bacteriol., 177, 4097-4104 (1995)) and plants (Mol. Gen. Genet., 224, 431-436 (1990), Plant Physiol., 100, 146-152 (1992), Plant Physiol., 103, 829-834 (1993), Plant Cell Physiol., 35, 1245-1249 (1994)) are known.

The presence of two types of enzymes, i.e. an inducible enzyme (gene name: adi) and a noninducible enzyme (gene name: speA) particularly in E. coli is known. When the environment where the microorganism lives is placed in an extremely acidic condition, that is, when acid stress is generated, the adi is urgently induced and expressed in a mechanism for transiently getting through the crisis, thus decarboxylating arginine to form basic agmatine with which the neutralization is carried out (J. Bacteriol., 177, 4097-4104 (1995), Appl. Environ. Microbiol., 62, 3094-3100 (1996), J. Bacteriol., 181, 3525-3535 (1999)). The same mechanismis also known with respect to other decarboxylases, for example, ornithinedecarboxylase (Biochem. Biophys. Res. Commun., 20, 697-702 (1965)) etc. The mechanism of induction and expression of adi (J. Bacteriol., 175, 1182-1186 (1993), J. Bacteriol., 176, 6769-6775 (1994), Lett. Appl. Microbiol., 24, 319-328 (1997)), the enzymatic properties thereof including enzyme purification and crystallization (J. Biol. Chem., 243, 1671-1677 (1968)), the gene cloning and structural analysis thereof (J. Bacteriol., 175, 1221-1234 (1993)) etc., have been studied mainly in the E. coli system.

However, even when the known microorganism producing arginine decarboxylase is cultured, the amount of the arginine decarboxylase gene expressed is very limited which makes it difficult to secure the amount of the enzyme necessary for industrial production of agmatine. That is, from a practical viewpoint, no report has been made on production of a significant amount of agmatine from arginine, using a microorganism which has highly expressed arginine decarboxylase as an enzyme catalyst.

This is because the amplification and expression of arginine decarboxylase gene in a large amount was considered unrealistic for the reason that agmatine is alkaline which upon being highly concentrated without neutralization, is fetal to the microorganism.

### SUMMARY OF THE INVENTION

It is an object of this invention to provide a method in which arginine decarboxylase is used at a practical level, whereby agmatine useful as an industrial intermediate material is efficiently produced from arginine produced relatively inexpensively.

The present inventors attempted to create a recombinant Escherichia coli microorganism by cloning the arginine decarboxylase gene (adi) from Escherichia coli (E. coli) and introducing it into E. coli. As a result of extensive study they found that arginine decarboxylase can be expressed in a large amount, thus arriving at this invention.

The method of producing agmatine by decarboxylating arginine according to one aspect of the present invention uses a recombinant that has expressed the amplified arginine decarboxylase gene, or arginine decarboxylase or an arginine decarboxylase-containing material obtained from the recombinant.

The method of producing arginine decarboxylase according to another aspect of the present invention comprises culturing in a medium a recombinant that has expressed the amplified arginine decarboxylase gene, and accumulating arginine decarboxylase in any one or more of the medium and cells.

Other objects and features of this invention will become apparent from the following description with reference to the accompanying drawings.

### BRIEF DESCRIPTION OF THE DRAWINGS

Fig. 1 is a flowchart which shows the process for producing a recombinant where the arginine decarboxylase gene has been amplified and expressed,
Fig. 2 is a flowchart which shows the process for constructing plasmids pTrcadi1, pTrcadi2, pTrcadi3 and pUCTrcadi, and
Fig. 3 is a flowchart which shows the process for constructing plasmid pMANΔspeB.

### DETAILED DESCRIPTIONS

Embodiments of the methods according to the present invention are described in detail below with reference to the accompanying drawings. The description shall be given in the following sequence:
[I] A recombinant that has amplified and expressed arginine decarboxylase gene, and
[II] A method of producing agmatine.

### [I] A recombinant that has amplified and expressed arginine decarboxylase gene:

Arginine decarboxylase catalyzes the reaction of forming agmatine and carbon dioxide from arginine which is one of amino acids. The presence of an arginine decarboxylase gene in the genus Escherichia including E. coli and the genus Salmonella (J. Bacteriol., 177, 4097-4104 (1995) and plants (Mol. Gen. Genet., 224, 431-436 (1990), Plant Physiol., 100, 146-152 (1992), Plant Physiol., 103, 829-834 (1993), Plant Cell Physiol., 35 1245-1249 (1994)) is known. However, it is known that even when these known microorganisms producing arginine decarboxylase are cultured, arginine decarboxylase is hardly accumulated.

An amplified expressed gene referred to herein means that an expression of the gene has been amplified, that is, an amount of expressed protein coded by the gene has been increased.

In this invention, a recombinant expressing an increased amount of the arginine decarboxylase is prepared to secure the amount of the enzyme necessary for the reaction of decarboxylating arginine.

To increase the amount of the arginine decarboxylase expressed, a regulatory region for the arginine decarboxylase gene may be modified. Modification of the regulatory region refers to, e.g. insertion of a new strong promoter, introduction of mutation in a promoter thereby enhancing the promoter function, or inactivation of, e.g. a repressor protein bound to the regulatory region, whereby the transcribed amount of the downstream arginine decarboxylase gene is increased.

To increase the expressed amount of the arginine decarboxylase, it is preferable that the arginine decarboxylase gene is ligated to a multicopy vector to construct a recombinant DNA, and the recombinant DNA is harbored in microorganisms. To create the microorganisms with which the expressed amount of the arginine decarboxylase is increased, a necessary gene region is amplified and obtained, for example, by amplification with PCR (polymerase chain reaction) on the basis of information of the known gene in arginine decarboxylase-producing microorganisms such as E. coli etc., and then carried on a vector such as a plasmid to transform host cells.

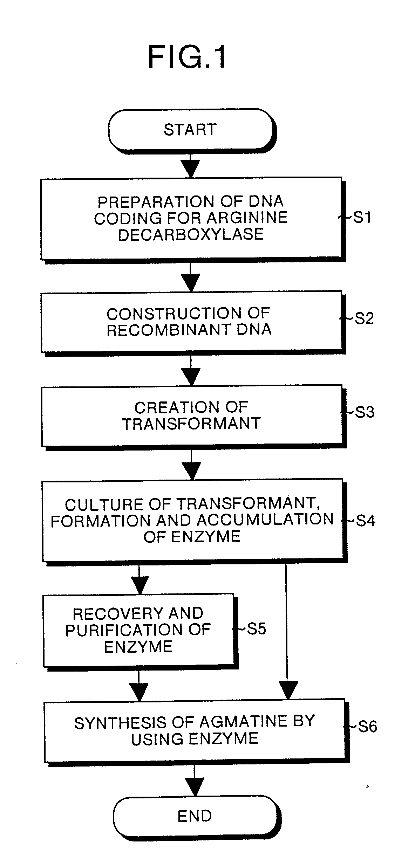

Fig. 1 is a flowchart of the process for producing a recombinant that has amplified and expressed arginine decarboxylase gene. First, a DNA coding for arginine decarboxylase in this invention is prepared (step S1).

The prepared arginine decarboxylase gene is then ligated to a vector DNA to prepare a recombinant DNA (step S2), and host cells are transformed to prepare a transformant (i.e. a recombinant) (step S3). Subsequently, the transformant is cultured in a medium, and arginine decarboxylase is formed and accumulated in the medium and/or cells (step S4).

At step S5, the formed arginine decarboxylase is recovered and purified, whereby arginine decarboxylase is produced in a large amount.

Further, the arginine decarboxylase prepared in step 5 or the medium in which arginine decarboxylase was accumulated therein in step 4 is used to decarboxylate arginine, whereby agmatine is produced in a large amount (step S6).

A method of producing a recombinant that has amplified and expressed arginine decarboxylase gene by recombinant DNA technique is described in detail in the following order.
(1) Preparation of an arginine decarboxylase gene
(2) Construction of a recombinant DNA
(3) Creation of a recombinant
(4) Formation and accumulation of arginine decarboxylase
(5) Recovery and purification of the arginine decarboxylase

### (1) Preparation of an arginine decarboxylase gene

The arginine decarboxylase gene can be obtained without any particular limitation from known microorganisms producing arginine decarboxylase, such as the genus Escherichia including E. coli and the genus Salmonella (J. Bacteriol., 177, 4097-4104 (1995)) and plants (Mol. Gen. Genet., 224, 431-436 (1990), Plant Physiol., 100, 146-152 (1992), Plant Physiol., 103, 829-834 (1993), Plant Cell Physiol., 35, 1245-1249 (1994)).

The presence of two types of enzymes, i.e. an inducible enzyme (gene name: adi) and a noninducible enzyme (gene name: speA) particularly in E. coli is known. When the environment where the microorganism lives is placed in an extremely acidic condition, that is, when acid stress is generated, the adi is urgently induced and expressed in a mechanism for transiently getting through the crisis, thus decarboxylating arginine to form basic agmatine with which the environment neutralization is carried out (J. Bacteriol., 177, 4097-4104 (1995), Appl. Environ. Microbiol., 62, 3094-3100 (1996), J. Bacteriol., 181, 3525-3535 (1999)).

As speAB, speA forms an operon, and speA coding for an enzyme forming agmatine and carbon dioxide from arginine and speB coding for an enzyme forming putrescine and urea from agmatine are known to be present (J. Bacteriol., 174, 758-764 (1992)). This enzyme system is noninducible, which together with ornithine decarboxylase decarboxylating ornithine to form putrescine, is understood to be a metabolic pathway necessary for the microorganism in biosynthesizing polyamines such as putrescine, spermidine etc. (Proc. Natl. Acad. Sci. U.S.A., 80, 5181-5184 (1983), J. Bacteriol., 173, 3615-3621 (1991), Int. J. Biochem., 26, 991-1001 (1994), J. Bacteriol., 180, 4278-4286 (1998), Microbiology, 145, 301-307 (1999)). The enzymatic and chemical properties of arginine decarboxylase encoded by speA and agmatinase encoded by speB and their gene structures have been studied in detail (J. Biol. Chem., 248, 1687-1695 (1973), Methods Enzymol., 94, 117-121 (1983), Gene, 30, 129-136 (1984), J. Bacteriol., 172, 4631-4640 (1990)). With respect to this enzyme system and the ornithine decarboxylase system in particular, mutant strains deficient in the ornithine decarboxylase gene and deficient in the agmatinase gene (speB) ,have been created to analyze the physiological functions of polyamines in the microorganisms (J. Bacteriol., 101, 725-730 (1970), J. Biol. Chem., 254, 12419-12426 (1979), Biochem., J., 234, 617-622 (1986), Proc. Natl. Acad. Sci. U.S.A., 84, 4423-4427 (1987)). Deficiency in both the enzymes may influence the growth of the microorganisms, but does not cause death and the physiological functions of polyamines in the microorganisms are not clarified (J. Bacteriol., 101, 731-737 (1970), J. Bacteriol., 113, 271-277 (1973), Adv. Polyamine Res., 4, 495-506 (1983), J. Bacteriol., 163, 522-527 (1985)). However, it is known that amines such as agmatine are volatile in the alkaline side, are stimulative to the skin and mucus and are absorbed into the body via the skin and mucus. Their toxicity to the microorganisms is not evident, but amines are alkaline, and when present at high concentration without neutralization, they are considered fatal to the microorganisms.

In this invention, the adi gene derived from E. coli is used particularly preferably as the arginine decarboxylase gene. speA is also a gene coding for arginine decarboxylase, but the arginine decarboxylase encoded by adi (J. Biol. Chem., 243, 1671-1677 (1968)) is advantageous over the arginine decarboxylase encoded by speA (J. Biol. Chem., 248, 1687-1695 (1973)) in respect of high specific activity etc.

To obtain the arginine decarboxylase gene, adi that is a gene coding for arginine decarboxylase may be cloned by PCR from chromosomal DNA in, e.g. W3110 strain (ATCC27325) of E. coli K12. Specifically, an about 30 base pairs of DNA molecule is synthesized on the basis of the sequence information of the known adi gene, and used as a probe which isolates the gene from an E. coli chromosomal gene library. The chromosomal DNA used may be derived from any strains as long as it is of E. coli.

A method of synthesizing the DNA molecule is disclosed in Tetrahedron Letters, 22, 1859 (1981). Further, the DNA can be synthesized by use of a synthesizer produced by Applied Biosystems. The DNA molecule can be utilized not only as a probe which isolates the E. coli-derived full-length adi gene from an E. coli chromosomal gene library but also as a primer which amplifies the E. coli-derived adi gene by PCR method.

The operation in PCR method is described by White, T. J. et al. in Trends Genet. 5, 185 (1989) etc. A method of preparing a chromosomal DNA and a method of isolating a desired DNA molecule from a gene library are described in Molecular Cloning, 2nd edition, Cold Spring Harbor press (1989) etc.

The adi gene used in this invention includes mutants due to genetic polymorphism. The genetic polymorphism refers to the phenomenon where the amino acid sequence of a protein is partially changed due to natural mutation on a gene.

To increase the activity of arginine decarboxylase, the structural gene itself for arginine decarboxylase may be mutated to increase the activity of the enzyme itself.

To mutate the gene, there are the following methods, a site specific mutation method (Kramer, W. and Frits, H. J., Methods Enzymol., 154, 350 (1987)), a recombinant PCR method (PCR Technology, Stockton Press (1989)), a method of chemically synthesizing a specific part of DNA, a method of treating with hydroxylamine, a method of treating a strain harboring the gene with UV irradiation or a method of chemically treating the gene with chemicals such as nitrosoguanidine or nitrous acid.

### (2) Construction of a recombinant DNA

The recombinant DNA in this invention is the one having the above-described arginine decarboxylase gene (adi etc.) ligated as a passenger to a vector such as plasmid or phage DNA.

The vector is preferably the so-called multicopy type, and a plasmid having an origin of replication derived from Col E1, for example a pUC series plasmid or a pBR 322 series plasmid, or a derivative thereof. As used herein, the "derivative" means that which has been modified to a plasmid by base substitution, deletion, insertion, addition or inversion. The modification mentioned here includes modification by mutagenesis with a mutagen or UV irradiation or by natural mutation. Besides, a transposon (Berg, D. E. and Berg, C. M., Bio/Technol., 1, 417 (1983)) and Mu phage (JP-A H2-109985) can also be used. The number of copies can also be increased by integrating the gene into the chromosome in the method of using a plasmid for homologous recombination.

To efficiently express the useful gene, lac promoter, trp promoter, tac promoter, trc promoter, PL promoter or other promoter functioning in microorganisms is preferably used.

To increase the amount of production, it is preferable that a transcription termination sequence, i.e. a terminator is ligated to a site downstream of the gene coding for the protein. This terminator includes a T7 terminator, a fd phage terminator, a T4 terminator, a terminator for tetracycline resistance gene, a terminator for E. coli trpA gene etc. Further, the amount transcribed of the gene may be increased by newly introducing an enhancer.

To select the transformant, the vector preferably has a marker such as ampicillin resistant gene etc., and as the plasmid, an expression vectors having a strong promoter, for example pUC series (product of Takara Shuzo Co., Ltd.), pPROK series (product of Clontech), pKK233-2 (product of Clontech) etc. are commercially available.

### (3) Creation of a recombinant

To induce of a recombinant having an increased amount of the arginine decarboxylase, host cells are transformed by introducing the recombinant DNA having the arginine decarboxylase gene (adi etc.) ligated to a vector such as plasmid or phase DNA.

As the host cells to be transformed, rganisms where the gene coding for arginine decarboxylase is expressed, for example, bacterial cells, actinomycetous cells, yeast cells, mold cells, plant cells, animal cells etc. can be used. Competent cells of preferably Enterobactera, more preferably Escherichia coli, particularly preferably E. coli JM109 etc. are used.

The recombinant DNA described above is used to transform host cells. As the method of transformation and the method of selecting transformants, methods described in Molecular Cloning, 2nd edition, Cold Spring Harbor press (1989) etc. can be applied.

### (4) Formation and accumulation of arginine decarboxylase

The recombinant transformed with the recombinant DNA containing the arginine decarboxylase gene (adi etc.), obtained in the method described above, is cultured to form and accumulate the desired enzyme. The method of culturing the recombinant that has acquired an ability to express the arginine decarboxylase gene at high levels is described below.

As the medium used, an LB medium (1 % Bacto-tryptone, 0.5 % Yeast extract, 1 % NaCl, 0.1 % Glucose, pH 7.0) is often used, but a usual medium containing a carbon source, a nitrogen source, inorganic ions and when necessary other organic components can be used.

As the carbon source, use can be made of sugars such as glucose, lactose, galactose, fructose, arabinose, maltose, xylose, trehalose, ribose, starch hydrolyzates etc., alcohols such as glycerol, mannitol, sorbitol etc., and organic acids such as gluconic acid, fumaric acid, citric acid, succinic acid etc.

As the nitrogen source, use can be made of inorganic ammonium salts such as ammonium sulfate, ammonium chloride, ammonium phosphate etc., organic nitrogen such as soybean hydrolyzates etc., ammonia gas, ammonia water etc. As organic trace nutrients, requisite substances such as various amino acids, vitamins such as vitamin B6 etc., nucleic acids such as RNA etc., or yeast extracts etc. are desirably contained in a suitable amount.

Besides, calcium phosphate, magnesium sulfate, iron ions, manganese ions etc. are added in a small amount when necessary.

Culture is conducted preferably under aerobic conditions for about 12 to 72 hours, and preferably the culture temperature is controlled at 20 to 45°C and the culture pH at 5 to 8. For pH adjustment, inorganic or organic, acidic or alkaline substances, ammonia gas etc. can be used.

When the expressed amount expressed of the arginine decarboxylase in the recombinant is greater than 10 % of the proteins in the microorganism, the arginine decarboxylase may have formed an inclusion body which may lower the activity of the arginine decarboxylase, but formation of the inclusion body can be disturbed by culturing the recombinant at 30°C or less.

Separation and recovery of the microorganism from the culture solution and preparation of the enzyme solution can be carried out usually by a combination of known methods such as centrifugation, ultrasonic disruption, an ion-exchange resin method, a precipitation method, etc.

### [II] A method of producing agmatine:

In the method of producing agmatine according to this invention, L-arginine is enzymatically decarboxylated using the arginine decarboxylase formed and accumulated in the recombinant that has amplified and expressed the arginine decarboxylase gene.

The method of allowing the arginine decarboxylase to act on L-arginine is not particularly limited, and for example, L-arginine may be added directly to the culture solution while the recombinant that has amplified and expressed the arginine decarboxylase gene is cultured, or the microorganism separated from the culture solution, the washed microorganism or the like may also be used. Alternatively, a cell extract obtained by disrupting or lysing the cell can be used as it is, or arginine decarboxylase may be recovered from the cell extract and used as a crude enzyme solution. Further, the arginine decarboxylase may be purified and used. That is, the purified enzyme and all the enzyme-containing materials can be used insofar as they are fractions having the activity of arginine decarboxylase. As used herein, the "enzyme-containing materials" may be those containing the enzyme and include, specifically, the cultured material, the cultured microorganism, the washed microorganism, the cell extract obtained by disrupting and lysing the cell, the crude enzyme, the purified enzyme, etc. From the viewpoint of reducing the cost for producing agmatine by simplifying the process, the method of reaction by adding the substrate directly to the culture solution is most preferable.

When the reaction of forming agmatine is allowed to proceedby adding L-arginine directly to the culture solution while culturing the transformant which has amplified and expressed the arginine decarboxylase gene, the reaction is conducted in a stationary state or with gentle stirring. Preferably, the reaction temperature is controlled at 10 to 60°C, preferably 25°C to 45°C, and the pH at 3 to 8, preferably pH 4.5 to 6. For the reaction, pyridoxal phosphate which is a coenzyme is preferably added. Insofar as the reaction proceeds, the substrate L-arginine may be added, and when necessary, can be reacted in a necessary amount for a necessary time.

When the reaction of forming agmatine is conducted using a crude enzyme solution, the cultured microorganism is recovered by the operation of centrifugation etc., and then the cells are disrupted or lysed to prepare a crude enzyme solution containing arginine decarboxylase. To disrupt the cells, a method such as ultrasonic disruption, French press disruption, glass bead disruption or the like can be used, while to lyse the cells, a method such as treatment with albumen lysozyme or peptidase or with a suitable combination of such is used. When the reaction of forming agmatine is conducted using a purified enzyme solution, the crude enzyme solution containing arginine decarboxylase is purified by usual techniques such as precipitation, filtration, column chromatography etc. In this instance, a method of purifying using an antibody to arginine decarboxylase can also be used.

When the reaction of forming agmatine is allowed to proceed by use of the crude enzyme solution or purified enzyme containing arginine decarboxylase, the reaction is allowed to proceed while a reaction solution containing the substrate L-arginine and the crude enzyme solution or purified enzyme is controlled at 10°C to 60°C, preferably 25°C to 45°C, and the pH at 3 to 8, preferably pH 4.5 to 6. For the reaction, pyridoxal phosphate which is a coenzyme is preferably added. Insofar as the reaction proceeds, the substrate L-arginine maybe added, and when necessary, can be reacted in a necessary amount for a necessary time.

### Example 1

### (Cloning and expression of the arginine decarboxylase gene (adi))

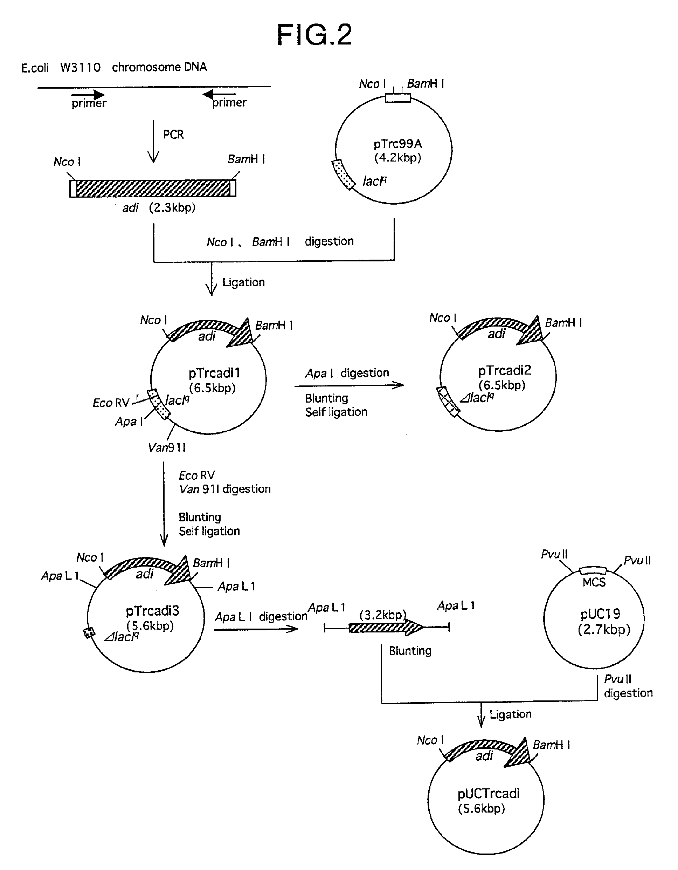

A pair of 30-mer and 28-mer primers GACCATGGCTAAAGTATTAATTGTTGAAAG (SEQ ID NO: 1) and CCGGATCCACGCCTTCAGCGGAATAGTG (SEQ ID NO: 2), and a pair of 28-mer and 28-mer primers CCCTGCAGATCAGTATCAGCCAAAAAAA (SEQ ID NO: 3) and CCGGATCCACGCCTTCAGCGGAATAGTG (SEQ ID NO: 2), prepared on the basis of information searched with the key word "adi" in a gene data bank (E. coli Gene Bank) and Pyrobest DNA polymerase (Takara Shuzo Co., Ltd.) were subjected to PCR (94 °C, 30 sec., 55°C, 1 min., 72°C, 2 min., 30 cycles, Gene Amp PCR System Model 19600 (Perkin Elmer)), whereby the adi structural gene region of about 2.3 kb fragment covering the region between ATG and the translation termination codon, or SD-ATG and the translation termination codon was amplified, and the former fragment was digested with NcoI and BamHI and inserted into between NcoI and BamHI sites in pTrc99A (Pharmacia Co., Ltd.). This expression plasmid is designated pTrcadi1 (Fig. 2). The latter fragment was digested with PstI and BamHI, and inserted into between PstI and BamHI sites in pUC19 (Takara Shuzo Co., Ltd.). This expression plasmid is designated pUCadi.

In the primers for PCR, the NcoI site is designed in SEQ ID NO:1, the BamHI site in SEQ ID NO:2, and the PstI site in SEQ ID NO:3. The cloned adi is expressed under the control of a trc promoter in pTrc99A vector or under the control of a lac promoter in pUC19 vector, and translated into arginine decarboxylase.

Further, recombinants derived from E. coli JM109 by transformation with these plasmids pTrcadi1 and pUCadi were cultured in LB liquid medium (1 % Bacto-tryptone, 0.5 % Yeast extract, 1 % NaCl, 0.1 % Glucose, pH 7.0) containing 50 mg/L ampicillin and 10 mg/L pyridoxine, and 1 mM IPTG (isopropyl-1-thio-β-D-galatoside) was added to induce expression, and the microorganisms were cultured for about 16 hours, and the cells were collected from 4 ml broth. These cells were suspended in 0.4 ml of 0.2 M sodium acetate buffer containing 1 % L-arginine·HCl and 0.02 % pyridoxal phosphate and incubated at 37°C for 1 hour. The cells were removed from the reaction solution by centrifugation, and as a result of analysis of the supernatant by HPLC, agmatine was formed in almost 100 % conversion in the pTrcadi1/JM109. In the pUCadi/JM109, the degree of conversion was about 20 %. When the recombinant cells not induced with IPTG were used, the degree of conversion for both was about 10 to 20 %. As the control, the host cell strain JM109 only, or the wild strain W3110, hardly caused conversion.

Further, pTrcadil/JM109 and pUCadi/JM109 induced with 1 mM IPTG were lysed with 1 % SDS solution and analyzed by SDS polyacrylamide electrophoresis (SDS-PAGE)-Coomassie Brilliant Blue (CBB) staining, and as a result, a significant band of about 85 kDa corresponding to the molecular weight of a subunit of arginine decarboxylase was found in the pTrcadi1/JM109 This accounted for about 10 % or more of the proteins in the reconbinant. In the pUCadi/JM109 and the pTrcadis/JM109 not induced with IPTG, a band that was significant as compared with that of the control could not be found at 85 kDa.

pTrcadi1/JM109, i.e. E. coli AJ13839 strain has been deposited as FERM P-18285 with International Patent Organism Depository (IPOD), National Institute of Advanced Industrial Science and Technology (AIST), Japan (Tsukuba City, Higashi 1-1-1, Ibaraki Prefecture, Japan) on April 2, 2001. Later, on Februaby 28, 2002, the same strain has been deposited as FERM BP-7931 based on Budapest convention.

The analysis conditions in HPLC are as follows.
Column: SUMIPAX PG-ODS 07-4625, 250x4.6 mm.
Mobile phase: acetonitrile/phosphate buffer (0.03 M NaH₂PO₄, pH 3.0) = 30/70.
Temperature: 40°C.
Flow rate: 1.0 ml/min.
Detector: 210 nm (UV).

### Example 2

### (Improvement of the arginine decarboxylase gene (adi) expression system)

In the expression plasmid pTrc99A carrying the arginine decarboxylase gene (adi), a gene coding for a repressor called lacI^{q} is carried on the same plasmid to usually suppress expression thereof under the control of the trc promoter. For expression, 1 to 2 mM IPTG is added during culture.

Accordingly, inactivation of the repressor was at tempted by partially processing the lacI^{q} gene of pTrcadil, in order to permit the arginine decarboxylase gene (adi) to be expressed even in the absence of IPTG. As the method for this, two methods, that is, a method of losing a restriction enzyme site ApaI site on lacI^{q} and a method of deleting between Van91I and EcoRV sites were used. That is, in the former method, pTrcadi1 was digested with ApaI, and the cohesive end of ApaI was blunt-ended with T4 DNA polymerase and self-ligated by T4 DNA ligase. The plasmid that turned to be incapable of cleavage with ApaI was designated pTrcadi2 (Fig. 2). In the latter method, pTrcadi1 was digested with Van91I and EcoRV, and the cohesive end of Van91I was blunt-ended with T4 DNA polymerase, and an about 6 kb DNA fragment cleaved with Van91I and EcoRV, from which small fragment had been removed, was self-ligated by T4 DNA ligase. The resultant plasmid was designated pTrcadi3 (Fig. 2).

pTrcadi2/JM109 and pTrcadi3/JM109 transformed pTrcadi2 and pTrcadi3 respectively were cultured in LB liquid medium containing 50 mg/L ampicillin and 10 mg/L pyridoxine for about 16 hours without adding IPTG, and the cells were collected from 4 ml broth. As a result of analysing of the degree of conversion of arginine into agmatine in the same reaction system as in Example 1, agmatine was formed in almost 100 % conversion for both instances.

Further, the pTrcadi2/JM109 and pTrcadi3/JM109 cells not induced with IPTG were lysed with 1 % SDS and analyzed by SDS-PAGE-Coomassie Brilliant Blue (CBB) staining, and as a result, a significant band of about 85 kDa corresponding to the molecular weight of a subunit of arginine decarboxylase was found in both pTrcadi2/JM109 and pTrcadi3/JM109, and accounted for about 5 % or more of the proteins in the cells.

### Example 3

### 1) Construction of a high-expression system for the arginine decarboxylase gene (adi)

The trc promoter and the adi gene region in pTrcadi3 were attempted to be carried on plasmid pUC19, in order to permit the arginine decarboxylase gene (adi) to be expressed at higher levels even in the absence of IPTG. It is said that plasmid pUC19 can attain the number of copies nearly 10 times as much as that by pTrc99A (whose replication function is derived from pBR322) . In the method therefor, pTrcadi3 was digested with restriction enzyme ApaLI (to generate 4 fragments), and the cohesive end of ApaLI was blunt-ended with T4 DNA polymerase, and an about 3.2 kb ApaLI fragment was separated and prepared. Then, pUC19 was digested with PvuII (to generate 2 fragments), and the resultant about 2.4 kb fragment was separated and prepared. The 3.2 kb and 2.4 kb fragments were ligated by T4 DNA ligase. The about 5.6 kb plasmid thus obtained was designated pUCTrcadi (Fig. 2).

E. coli JM109 transformed with pUCTrcadi (pUCTrcadi/JM109) was used and cultured in LB liquid medium containing 50 mg/L ampicillin and 10 mg/L pyridoxine for about 16 hours without adding IPTG, and the cells were collected from 0.4 ml broth. As a result of analysis of the degree of conversion of arginine into agmatine in the same reaction system as in Example 1, agmatine was formed in almost 100 % conversion. This result indicated almost the same activity as in the method of using the pTrcadil/JM109 (strain) induced with IPTG in Example 1.

Further, the cultured cells of the pUCTrcadi/JM109 not induced with IPTG were lysed with 1 % SDS solution and analyzed by SDS-PAGE-Coomassie Brilliant Blue (CBB) staining, and as a result, a significant band of about 85 kDa corresponding to the molecular weight of a subunit of arginine decarboxylase was found in the pUCTrcadi/JM109 strain. This accounted for 20 % or more of the proteins in the cell, and formation of the inclusion body was observed in some cells. Formation of the inclusion body can be inhibited by culturing the pUCTrcadi/JM109 at a temperature of 30°C or less, and the arginine decarboxylase could be confirmed to account for about 20 % of the proteins in this cell.

### Example 4

### 1) Cloning of the agmatinase gene (speB) and breeding of a microbial strain containing the gene destroyed

A pair of 28-mer and 28-mer primers CCGAATTCACGTCCATCCCAACAATGTT (SEQ ID NO: 4) and CCGCATGCGGCGATGCGTGTGAAGAAAA (SEQ ID NO: 5) prepared on the basis of information searched with the key word "speAB" in a gene data bank (E. coli Gene Bank) and Pyrobest DNA polymerase (Takara Shuzo Co., Ltd.) were subjected to PCR (94°C, 30 sec., 55°C, 1 min., 72°C, 2 min., 30 cycles, Gene Amp PCR System Model 9600 (Perkin Elmer)), whereby an about 1.9 kb fragment of a partial gene region in speAB was amplified, and this fragment was digested with EcoRI and SphI and inserted into between EcoRI site and SphI site in pUC19 (Takara Shuzo Co., Ltd.). This expression plasmid was designated pUCspeAB. In the primers for PCR, the EcoRI site is designed in SEQ ID NO: 4 and the SphI site in SEQ ID NO: 5.

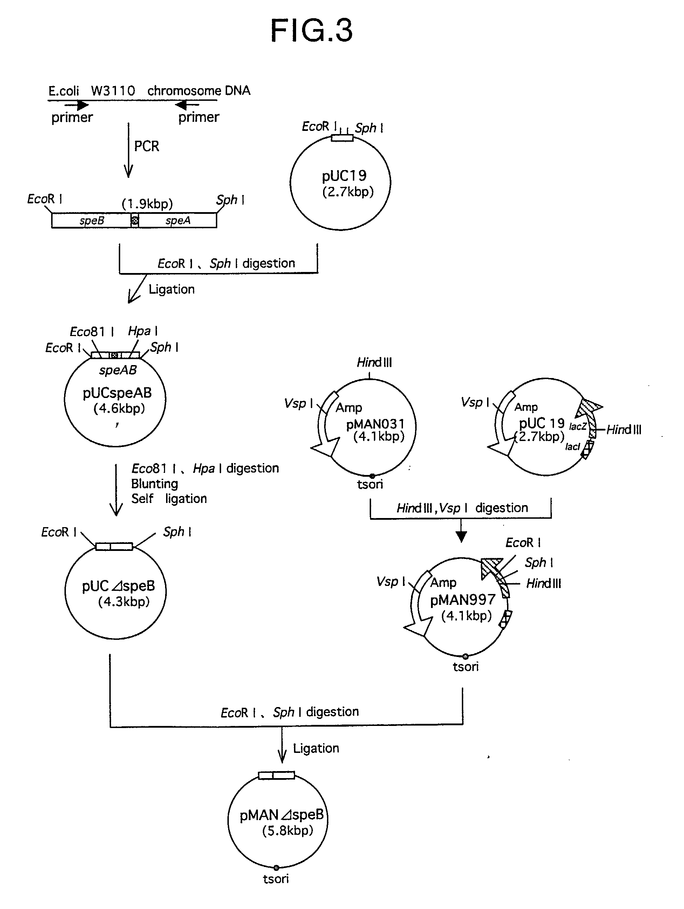

Then, inactivation of the function of speB was attempted by processing a part of the speAB gene. In the method therefor, the region between restriction enzymes sites Eco81I and HpaI sites on speAB were deleted. That is, pUCspeAB was digested with Eco81I and HpaI, and the cohesive end of Eco81I was blunt-ended with T4 DNA polymerase, and then an about 4.3 kb DNA fragment cleaved with Eco81I and HpaI, from which a small fragment (about 260 bp) had been removed, was self-ligated by T4 DNA ligase. The resultant plasmid was designated pUCΔspeB (Fig. 3). Then, pUCΔspeB was cleaved with EcoRI and SphI, to give an about 1.7 kb fragment containing speAB, and this fragment was inserted into between EcoRI site and SphI site in pMAN997 (WO99/03988) i.e. a homologous recombination vector having a temperature sensitive origin of replication (tsori), to obtain the desired plasmid. This plasmid was designated pMANΔspeB (Fig. 3).

pMAN997 was constructed by exchanging a HindIII-VspI fragment not containing the side of tsori in pMAN031 (S. Matsuyama, et al., J. Bacteriol., 162, 1196-1202 (1985)) with a HindIII-VspI fragment not containing the side of ori in pUC19.

Then, pMANΔspeB was transformed into E. coli W3110 (wild strain), and the resultant transformant was plated onto LB agar medium containing 50 mg/L ampicillin, and then cultured at 30°C overnight.

Then, these cultured transformants were plated onto LB agar medium containing 50 mg/L ampicillin so as to form each single colony, to obtain colonies growing at 42°C. The operation of obtaining each single colony growing at 42 °C was repeated to select a clone having the whole of the plasmid integrated via homologous recombination into the chromosome thereof. It was confirmed that this clone does not have the plasmid in its cell sap. Then, some of the clones were plated onto LB agar medium, then cultured at 30°C overnight, successively inoculated into 3 ml LB liquid medium/test tube, and subjected to shake culture at 42°C for 3 to 4 hours. This culture solution was suitably diluted (10⁻⁵ to 10⁻⁶) so as to obtain each single colony, plated onto LB agar medium and cultured at 42°C overnight to obtain colonies. From the appeared colonies, 100 colonies were picked up at random and allowed to grow in both of LB agar medium only and LB agar medium containing 50 mg/L ampicillin, and ampicillin-sensitive clones growing in the LB agar medium only were selected. Out of these sensitive clones, several clones were used to prepare chromosomal DNA which was then subjected to PCR (under the same conditions as above) with the primers in SEQ ID NOS: 4 & 5, to amplify an about 1.7 kb or about 2 kb fragment of speAB. It was confirmed that about 1.7 kb fragment cannot be cleaved with Eco81I or HpaI. A strain having, in its chromosome, the ΔspeB gene in which the about 1.7 kb fragment had been detected was obtained as a speB-deficient strain (speB⁻) incapable of expressing agmatinase.

### 2) Evaluation of production of agmatine by using the speB-deficient strain

The arginine decarboxylase gene (adi)-expressing plasmid pTrcadi2 constructed in Example 2 was introduced into the speB-deficient strain to create pTrcadi2/speB⁻ strain.' Using this transformant, it was cultured in LB liquid medium containing 50 mg/L ampicillin and 10 mg/L pyridoxine, and cultured in the absence of IPTG for about 16 hours, and then the cells were collected from 4 ml broth. As a result of analysis of the degree of conversion of arginine into agmatine in the same reaction system as in Example 1, agmatine was formed in almost 100 % conversion. Further, formation of putrescine which had been detected in a small amount in speB⁺ strain (JM109) by analysis in thin layer chromatography (conditions: detection with staining with ninhydrin after separation with a solvent system of chloroform/methanol/ammonia water = 2/4/3) disappeared in this speB⁻ strain. This is probably because formation of putrescine from agmatine, catalyzed by agmatinase encoded by speB, disappears due to deficiency in speB. By blocking this agmatine decomposition system, there occurs the advantage for inhibiting a reduction in the yield of agmatine and for preventing contamination of agmatine with the same amine, putrescine.

Further, the pTrcadi2/speB⁻ strain not induced with IPTG was lysed with 1 % SDS solution and analyzed with by SDS-PAGE-Coomassie Brilliant Blue (CBB) staining, and as a result, a significant band of about 85 kDa corresponding to the molecular weight of a subunit of arginine decarboxylase was found. This accounted for about 5 % of the proteins in the cells, but the feature of instable maintenance of the plasmid during culture was also found.

According to this invention, the arginine decarboxylase gene could be expressed stably in a large amount in hosts such as E. coli. As a result, such transformants could be used for efficiently producing agmatine useful as an industrial intermediate material, from arginine produced relatively inexpensively.

Further, agmatine obtained by the production method of this invention is used as an intermediate for production of acyl agmatine, whereby the complicated chemical synthesis process necessary for synthesis of acyl agmatine can be partially simplified to reduce the cost of acyl agmatine useful as a surfactant.

Although the invention has been described with respect to a specific embodiment for a complete and clear disclosure, the appended claims are not to be thus limited but are to be construed as embodying all modifications and alternative constructions that may occur to one skilled in the art which fairly fall within the basic teaching herein set forth.

## Claims

1. A method of producing agmatine by decarboxylating arginine, the method comprising using a recombinant that has amplified and expressed arginine decarboxylase gene, or arginine decarboxylase or an arginine decarboxylase-containing material obtained from the recombinant.

2. The method of producing agmatine according to claim 1, wherein the recombinant that has amplified and expressed arginine decarboxylase gene is used.

3. The method of producing agmatine according to claim 1, wherein the amount of arginine decarboxylase expressed in the recombinant is greater than 5 % of the proteins in the recombinant.

4. The method of producing agmatine according to claim 1, wherein the arginine decarboxylase gene is an adi gene derived from Escherichia coli.

5. The method of producing agmatine according to claim 1, wherein the recombinant is a microorganism transformed using a recombinant DNA obtained by ligating a vector DNA derived from a pUC series plasmid, a pBR322 series plasmid or a derivative thereof to the arginine decarboxylase gene.

6. The method of producing agmatine according to claim 5, wherein the recombinant DNA harbors a trc promoter.

7. The method of producing agmatine according to claim 1, wherein the recombinant has Escherichia coli as a host.

8. The method of producing agmatine according to claim 7, wherein the recombinant is FERM BP-7931 strain.

9. The method of producing agmatine according to claim 7, wherein the recombinant is Escherichia coli deficient in agmatinase catalyzing conversion of agmatine into putrescine.

10. The method of producing agmatine according to claim 1, wherein the pH in the reaction system for the reaction of converting arginine into agmatine is regulated to be less than pH 6.

11. The method of producing agmatine according to claim 1, wherein pyridoxal phosphate is added to the reaction system for the reaction of converting arginine into agmatine.

12. The method of producing agmatine according to claim 1, comprising:
culturing a recombinant that has amplified and expressed arginine decarboxylase gene at a temperature of less than 30 degree centigrade thereby accumulating arginine decarboxylase in the recombinant; and
decarboxylating arginine using the recombinant that has amplified and expressed arginine decarboxylase gene, or arginine decarboxylase or an arginine decarboxylase-containing material obtained from the recombinant to produce agmatine.

13. A method of producing arginine decarboxylase, wherein a recombinant that has amplified and expressed arginine decarboxylase gene is cultured in a medium, and arginine decarboxylase is accumulated in any one or more of the medium and cells.
